(19) Europäisches Patentamt / European Patent Office / Office européen des brevets

(11) **EP 2 770 070 A1**

(12) **EUROPEAN PATENT APPLICATION**
published in accordance with Art. 153(4) EPC

(43) Date of publication:
**27.08.2014 Bulletin 2014/35**

(21) Application number: **12842425.6**

(22) Date of filing: **18.10.2012**

(51) Int Cl.:
*C22C 19/07* [(2006.01)]     *B22F 3/105* [(2006.01)]
*B22F 3/16* [(2006.01)]     *C22C 1/04* [(2006.01)]

(86) International application number:
**PCT/JP2012/076999**

(87) International publication number:
**WO 2013/058339 (25.04.2013 Gazette 2013/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.10.2011   JP 2011231703**

(71) Applicants:
• **Kyocera Medical Corporation**
  **Osaka-shi, Osaka 532-0003 (JP)**
• **National University Corporation**
  **Tokyo Medical and Dental University**
  **Bunkyo-ku**
  **Tokyo 113-8510 (JP)**

(72) Inventors:
• **NOMURA, Naoyuki**
  **Tokyo 113-8510 (JP)**
• **HANAWA, Takao**
  **Tokyo 113-8510 (JP)**

(74) Representative: **Müller-Boré & Partner**
**Patentanwälte PartG mbB**
**Friedenheimer Brücke 21**
**80639 München (DE)**

(54) **IMPLANT Co-Cr-Mo ALLOY**

(57)     The present invention is to provide a Co-Cr-Mo alloy excellent in mechanical properties such as yield strength and tensile strength. The present invention is a biocompatible Co-Cr-Mo alloy comprising, in mass%, more than 30% and not more than 36% of Cr, 5 to 8% of Mo, 0.20 to 0.65% of N, and the balance consisting of Co and inevitable impurities, and produced by layered manufacturing. The biocompatible Co-Cr-Mo alloy of the present invention preferably has a solidification structure of a dendrite structure and the primary arm spacing of the dendrite structure is not more than 5 $\mu$m.

**EP 2 770 070 A1**

**Description**

TECHNICAL FIELD

The present invention relates to a biocompatible Co alloy, and particularly to a Co-Cr-Mo alloy excellent in mechanical properties, particularly, proof stress, tensile strength, and the like.

BACKGROUND ART

Co-Cr-Mo alloys have been used widely all over the world as a biocompatible material and, for example, a Co-28% Cr-6% Mo alloy (casting material) standardized as ASTM F75 has been known. However, the cast material standardized as ASTM F75 is difficult to sufficiently suppress solidification defects and segregation as it is and there is room for improvement of strength and ductility.

In order to solve such problems of the material standardized as ASTM F75, for example, Patent Document 1 proposes a casting material of a Co-Cr-Mo alloy with increased contents of Cr and nitrogen as compared with the above-mentioned standardized material.

PRIOR ART DOCUMENT

PATENT DOCUMENT

Patent Document 1: JP-A-2009-114477

SUMMARY OF THE INVENTION

PROBLEMS TO BE SOLVED BY THE INVENTION

According to the above-mentioned Patent Document 1, it is described that a cast Co alloy containing, in mass%, more than 30% and not more than 36% of Cr, 5 to 8 % of Mo, and 0.20 to 0.65% of N has improved yield strength, tensile strength, and elongation as compared with the material standardized as ASTM F75.

In Patent Document 1, an ingot of a Co-Cr-Mo type alloy is produced by metal mold casting and mechanical properties thereof are measured. However, according to the investigations carried out by the inventors of the present invention, in the case where the Co-Cr-Mo type alloy described in Patent Document 1 is produced by sand mold casting which is employed most practically in biomaterials, it is supposed that the mechanical properties as disclosed in Cited Document 1 cannot necessarily be realized. Here, from the viewpoint of particularly high strength, it is generally effective to carry out hot processing such as hot rolling or hot forging. However, the Co-Cr-Mo alloy with the composition described in Patent Document 1 is poor in the hot processability and difficult to have high strength by hot processing and thus it is difficult to obtain a Co-Cr-Mo alloy excellent in yield strength, tensile strength and the like.

Accordingly, the present invention aims to provide a Co-Cr-Mo alloy excellent in mechanical properties such as yield strength (0.2% proof stress) and tensile strength.

MEANS FOR SOLVING THE PROBLEMS

The present invention which has achieved the above-mentioned problem is a biocompatible Co-Cr-Mo alloy comprising, in mass%,
more than 30% and not more than 36% of Cr,
5 to 8% of Mo,
0.20 to 0.65% of N, and the balance consisting of Co and inevitable impurities, and
produced by layered manufacturing.

The biocompatible Co-Cr-Mo alloy of the present invention preferably has a solidification structure of a dendrite structure and the primary arm spacing of the dendrite structure is not more than 5 $\mu$m. The biocompatible Co-Cr-Mo alloy of the present invention has the 0.2% proof stress of not less than 700 MPa and the tensile strength of not less than 980 MPa, for example.

The present invention also comprises a powder to be used for producing the biocompatible Co-Cr-Mo alloy. The powder has a gist for having a particle diameter of not more than 100 $\mu$m.

The present invention also comprises a process for producing a Co-Cr-Mo alloy wherein the Co-Cr-Mo alloy having the above-described chemical composition is layered-manufactured.

In the production process of the present invention, layered manufacturing is preferably carried out by irradiating laser of an output power of not less than 50W and adjusting the scan pitch in the plane direction to be not less than 0.1mm. As well, the scan pitch in the plane direction means a irradiation spacing of laser.

EFFECTS OF THE INVENTION

According to the present invention, a Co-Cr-Mo alloy excellent in 0.2% proof stress, tensile strength, and elongation is provided since a Co-Cr-Mo alloy with high Cr and high N is produced by layered manufacturing.

BRIEF DESCRIPTION OF THE DRAWINGS

[Fig. 1] Fig. 1 is a graph illustrating a relationship between the scan pitch in the plane direction and 0.2% proof stress.
[Fig. 2] Fig. 2 is a graph illustrating a relationship between the output power of laser, and tensile strength and elongation.
[Fig. 3] Fig. 3 is an optical microscope photograph obtained by observing the structure of a Co alloy produced in the Examples described later.

MODE FOR CARRYING OUT THE INVENTION

The inventors of the present invention repeatedly made investigations to provide a Co-Cr-Mo alloy excellent in mechanical properties such as yield strength and tensile strength and found that a Co-Cr-Mo alloy excellent in 0.2% proof stress, tensile strength and elongation is obtained by powdering a Co-Cr-Mo alloy with a composition described in Patent Document 1 and carrying out layered manufacturing of the powder, and the finding now lead to completion of the present invention. Hereinafter, the alloy composition of the present invention and layered manufacturing will be described in order.

A Co-Cr-Mo alloy of the present invention contains, in mass%, more than 30% and not more than 36% of Cr, 5 to 8 % of Mo, and 0.20 to 0.65% of N.

Cr is an element indispensable for surely attaining corrosion resistance. In the present invention, the mechanical properties are further improved and the amount of dissolved N is increased by adjusting the Cr amount to be more than 30% (it means mass%, hereinafter the same in the chemical composition) . The Cr amount is preferably not less than 31% and more preferably not less than 32%. On the other hand, if the Cr amount is excessive, the mechanical properties such as tensile strength and elongation are rather deteriorated. Therefore, the Cr amount is determined to be not more than 36% in the present invention. The Cr amount is preferably not more than 35% and more preferably not more than 34%.

Mo is an element effective for improvement of corrosion resistance and wear resistance. Therefore, the Mo amount is determined to be not less than 5%. The Mo amount is preferably not less than 6%. On the other hand, if the Mo amount is excessive, it results in deterioration of processability. Therefore, the Mo amount is determined to be not more than 8%. The Mo amount is preferably not more than 7%.

N is an element for forming a stable γ phase and having an action of improving ductility. N is an element also having an action of improving the 0.2% proof stress. Therefore, the N amount is determined to be not less than 0.20% in the present invention. The N amount is preferably not less than 0.25% and more preferably not less than 0.30%. On the other hand, if the N amount is excessive, $Cr_2N$ is precipitated and mechanical properties are deteriorated. Therefore, the N amount is determined to be not more than 0.65%. The N amount is preferably not more than 0.60% and more preferably not more than 0.55%.

The composition of the Co alloy of the present invention is as described above and the balance is made up by substantially Co. Co is an element having corrosion resistance and wear resistance. In addition, it is naturally allowed that the Co alloy of the present invention contain inevitable impurities brought into depending on the raw materials, resources, the situation of production equipment, and the like. Further, in the present invention, if necessary, the Co alloy may contain at least one element selected from the group consisting of not more than 0.2% of C, not more than 1.00% of Ni, not more than 1.00% of Si, and not more than 1.00% of Mn.

The Co alloy of the present invention is characterized by being produced by layered manufacturing. The Co alloy produced by layered manufacturing has a fine solidification structure and in general, the solidification structure is a dendrite structure, and the primary arm spacing of the dendrite structure is, for example, not more than 5 μm and preferably not more than 1.5 μm. The lower limit of the arm spacing is generally around 0.5 μm.

Next, the layered manufacturing will be described. The layered manufacturing is described in, for example, Japanese Patent No. 4054075, and is a method for producing a compact by spreading a material powder in a layer form, melting and thereafter solidifying the material powder by irradiating the powder with electromagnetic radiation such as laser beam or corpuscular radiation such as electron beam. In the present invention, since the Co alloy with the above-mentioned composition is obtained by layered manufacturing, it is made possible to provide the Co alloy excellent in elongation in addition to 0.2% proof stress, tensile strength. Further, by layered manufacturing, the irradiation pattern of the radial rays is precisely controlled and therefore, shaping into a complicated shape is made possible.

According to the investigations made by the inventors of the present invention, there is a correlation between the mechanical properties (0.2% proof stress, tensile strength, and elongation) of a Co alloy and the condition of the layered manufacturing, and particularly the 0.2% proof stress is more improved by widening the scan pitch in the plane direction and particularly the tensile strength and elongation is more improved by increasing the output power of the radial rays. Concretely, the scan pitch in the plane direction is preferably adjusted to be not less than 0.1 mm, more preferably not less than 0.2 mm, and even more preferably not less than 0.3 mm. The upper limit of the scan pitch in the plane direction is, for example, not more than 0.5 mm for preventing pore formation. The output power of the radial rays is preferably not less than 50 W, more preferably not less than 100 W, and even more preferably not less than 150 W. Although it depends on the apparatus for outputting the radial rays, the upper limit of the output power of the radial rays is, for example, not more than 400 W.

Other production conditions for the layered manufacturing may be set properly and, for example, the radiation diameter (e.g., radius of circle) of radial rays may be adjusted to be about 0.1 to 1 mm; the distance from the radial ray source to the material powder may be adjusted to be about 100 to 10000 mm; and the layer thickness (thickness of one layer of the powder) may be adjusted to be about 0.01 to 0.1 mm. The atmosphere at the time of the layered manufacturing is also not particularly limited, and it is preferable to carry out the layered manufacturing in an atmosphere of an inert gas such as argon gas or nitrogen gas.

The material powder used for the layered manufacturing can be prepared by an atomization method (water atomization method or gas atomization method), a rotating electrode process, a ball mill method, and the like. It is preferable that the powder prepared by the above-mentioned method is sieved if necessary to have a particle diameter of not more than 100 $\mu$m (preferably not more than 70 $\mu$m, and more preferably not more than 50 $\mu$m). In addition, the particle diameter of the material powder is generally not below 5 $\mu$m. In this description, the particle diameter means the maximum diameter.

The type of radial rays in the layered manufacturing is not particularly limited, but laser of electromagnetic radiation is employed in the present invention. Examples of the type of laser include YAG laser, excimer laser, semi-conductor laser, and $CO_2$ laser.

The Co alloy of the present invention produced by layered-manufacturing the Co alloy with the above-mentioned composition is excellent in mechanical properties such as 0.2% proof stress, tensile strength, and elongation. The 0.2% proof stress is, for example, not less than 700 MPa (preferably not less than 730 MPa and more preferably not less than 760 MPa) and the tensile strength is, for example, not less than 980 MPa (preferably not less than 1000 MPa and more preferably not less than 1020 MPa). The elongation (total elongation) is, for example, not less than 8.0% (preferably not less than 10.0% and more preferably not less than 15.0%).

The structure of the Co alloy of the present invention is generally a dendrite structure. Because of the effect of the anisotropy of the structure, the mechanical properties of the Co alloy are different depending on the directions (directions to the layer direction). Concretely, the 0.2% proof stress and tensile strength show higher values in the direction perpendicular to the layered direction and the elongation shows a higher value in the direction parallel to the layered direction. Consequently, it is preferable to produce a biocompatible material while properly adjusting the direction to which a load is mainly applied at the time of use of the material and the layered direction, in accordance with the use application of the biocompatible material. Concretely, in the case of a use application in which the 0.2% proof stress and tensile strength are mainly required, it is proper to produce the Co alloy of the present invention by layered manufacturing in a manner that the load direction at the time of use of the biocompatible material and the layered direction are perpendicular to each other. Also, in the case of a use application in which elongation is mainly required, it is proper to produce the Co alloy of the present invention by layered manufacturing in a manner that the load direction at the time of use of the biocompatible material and the layered direction are parallel to each other.

This application claims the benefit of priority based on Japanese Patent Application No. 2011-231703 filed on October 21, 2011. All the contents of Japanese Patent Application No. 2011-231703 filed on October 21, 2011 are incorporated herein by reference.

EXAMPLES

Hereinafter, the present invention will be described more concretely with reference to examples. The present invention is not limited to the following examples and various modifications can be appropriately made without departing from the gist of the invention as defined above or hereinafter, all of which fall within the technical scope of the present invention.

A molten Co alloy with the chemical composition described in Table 1 was prepared and a Co alloy powder was produced by a water atomization method. Thereafter, the powder was sieved to produce a Co alloy powder with a particle diameter of not more than 45 μm.

Each Co alloy in form of a dumbbell type tensile test specimen according to JIS T6115, the dentistry casting cobalt-chromium alloy, was produced from the above-mentioned powder by a layered manufacturing apparatus (EOSINT M250 xtended) with the laser output power and scan pitch (in plane direction) as described in Table1 (test Nos. 1 to 7, 9 and 11 to 15). Test Nos. 1 to 5, 9, and 11 to 14 were layered-manufactured in a manner that the tensile test direction and the layered direction were to be parallel to each other and test Nos. 6, 7, and 15 were layered-manufactured in a manner that the tensile test direction and the layered direction were to be perpendicular to each other. The layered manufacturing was carried out in an argon atmosphere, the irradiation diameter of laser was 0.4 mm (400 microns), and the layered thickness was 0.05 mm.

Each specimen was subjected to measurement of 0.2% proof stress, tensile strength, and elongation (total elongation) according to JIS T6115. The number of tests for each test No. was 3.

For comparison, Table 1 also shows the results of a specimen (test No. 8) produced from Co-33 mass% Cr-5 mass% Mo-0.34 mass% N satisfying the composition of the present invention by a centrifugal casting method using a room temperature sand mold and a specimen (test No. 10) produced from Co-29 mass% Cr-6 mass% Mo, a material standardized as ASTM F75, by a centrifugal casting method using a room temperature sand mold. In Table 1, in order to describe that the samples were produced by the centrifugal casting method, "As-Cast" is written in the column of laser output power.

[Table 1]

| Test No. | Compositon of alloy (mass%) | Output power of laser (W) | Scan pitch[※1] (mm) | Tensile direction [※2] | 0.2% proof stress (MPa) | Tensile strength (MPa) | Elongation El (%) |
|---|---|---|---|---|---|---|---|
| 1 | Co-33Cr-5Mo-0.34N | 100 | 0.1 | parallel | 789 | 1017 | 15.9 |
| 2 | | 150 | 0.1 | parallel | 760 | 1031 | 18.8 |
| 3 | | 200 | 0.1 | parallel | 744 | 1040 | 22.4 |
| 4 | | 200 | 0.2 | parallel | 814 | 1030 | 15.9 |
| 5 | | 200 | 0.3 | parallel | 829 | 1050 | 16.7 |
| 6 | | 200 | 0.1 | perpendicular | 900 | 1195 | 11.7 |
| 7 | | 200 | 0.2 | perpendicular | 863 | 1123 | 8.6 |
| 8 | | As-Cast | - | - | 606 | 910 | 14.2 |
| 9 | Co-29Cr-6Mo | 200 | 0.1 | parallel | 538 | 949 | 16.4 |
| 10 | | As-Cast | - | - | 223 | 500 | 3.2 |
| 11 | Co-33Cr-5Mo-0.41N | 65 | 0.1 | parallel | 862 | 1117 | 21.8 |
| 12 | | 98 | 0.1 | parallel | 845 | 1123 | 24.8 |
| 13 | | 130 | 0.1 | parallel | 819 | 1120 | 26.2 |
| 14 | | 130 | 0.2 | parallel | 856 | 1110 | 18.3 |
| 15 | | 130 | 0.1 | perpendicular | 996 | 1328 | 13.2 |

[※1] It means the scan pitch in the plane direction.

[※1] It measns the tensile direction to the layered direction.

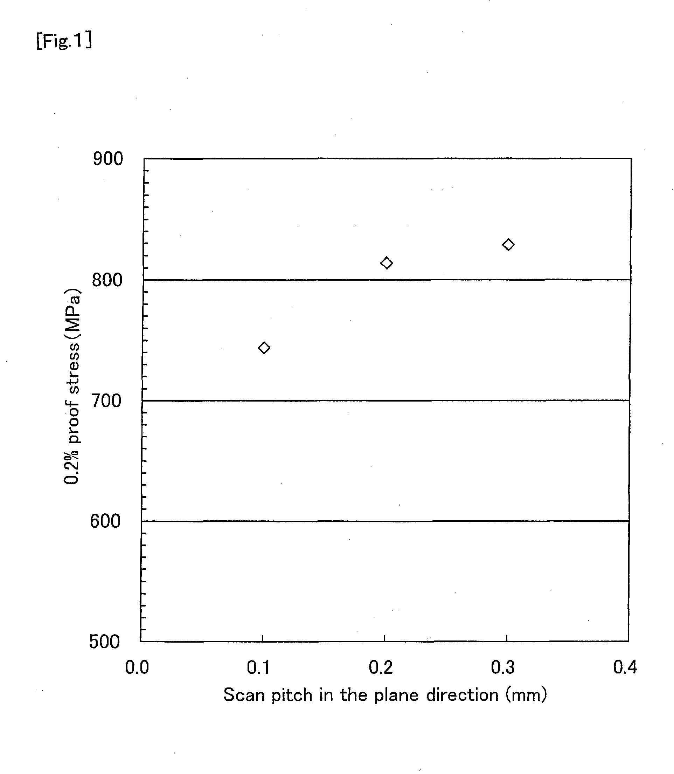

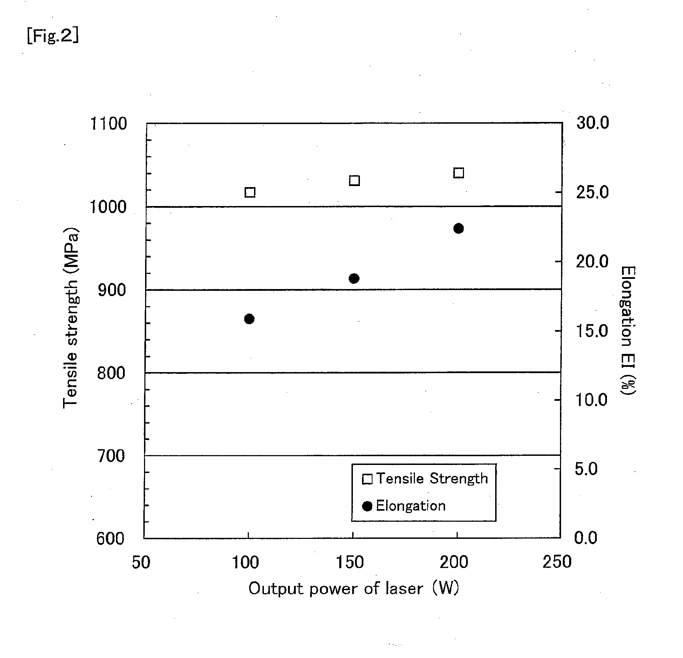

According to Table 1, all specimens of test Nos. 1 to 7 and 11 to 15, which are Co alloys of the present invention, were excellent in mechanical properties of 0.2% proof stress, tensile strength, and elongation. Further, from the results of these tests, the effects on the mechanical properties given by the scan pitch and the output power of the radial rays were made clear. Fig. 1 is a graph illustrating a relationship between the scan pitch in the plane direction and 0.2% proof stress, and Fig. 2 is a graph illustrating a relationship between the output power of the radial rays (output power of laser in the examples) and tensile strength and elongation. According to Fig. 1 and Fig. 2, it can be understood that the 0.2% proof stress is improved as the scan pitch becomes wider and that the tensile strength and elongation are improved as the output power of laser becomes higher.

Comparing test No. 3 with test No. 6, it can be understood that elongation is excellent in the case where the tensile direction is parallel to the layered direction and that the 0.2% proof stress and tensile strength are excellent in the case where the tensile direction is perpendicular to the layered direction. The same tendency can be seen in the comparison of test No. 4 with test No. 7 or of test No. 13 with test No. 15.

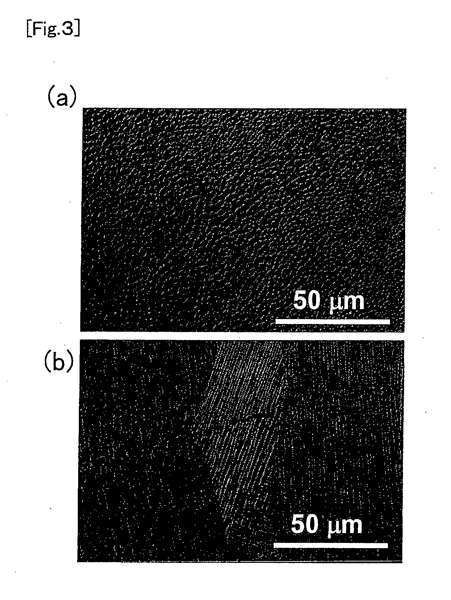

Further, Fig. 3 shows a photograph of a structure of the specimen of test No. 3 observed by an optical microscope. Fig. 3(a) is a photograph of a structure of a cross section perpendicular to the layered direction, and Fig. 3(b) is a photograph of a structure of a cross section parallel to the layered direction. According to Fig. 3, it can be understood that a fine dendrite structure is formed.

When the arm spacing of primary arms of the dendrite structure for the test No. 3 was measured by measuring the number n of times the dendrite interface crossed a certain reference length L and carrying out calculation according to L/(n - 1), it was 1.5 $\mu$m. The arm spacings of primary arms of the dendrite structure for the test Nos. 1, 2, 4 to 7, and 11 to 15 were all not more than 5 $\mu$m, but the arm spacings of primary arms of the dendrite structure for the test Nos. 8 and 10, which are out of the scope of the present invention, exceeded 5 $\mu$m because the specimens were produced by a centrifugal casting method.

INDUSTIAL APPLICABILITY

The Co-Cr-Mo alloy of the present invention can be used suitably as an biocompatible material, for example, for dentistry, orthopedics, and the like.

**Claims**

1. A biocompatible Co-Cr-Mo alloy comprising, in mass%,
   more than 30% and not more than 36% of Cr,
   5 to 8% of Mo,
   0.20 to 0.65% of N, and the balance consisting of Co and inevitable impurities, and
   produced by layered manufacturing.

2. The biocompatible Co-Cr-Mo alloy according to claim 1, wherein a solidification structure is a dendrite structure, and the primary arm spacing of the dendrite structure is not more than 5 $\mu$m.

3. The biocompatible Co-Cr-Mo alloy according to claim 1 or 2, wherein the 0.2% proof stress is not less than 700 MPa and the tensile strength is not less than 980 MPa.

4. A biocompatible Co-Cr-Mo alloy powder used for producing the biocompatible Co-Cr-Mo alloy according to any one of claims 1 to 3, having a particle diameter of not more than 100 $\mu$m.

5. A process for producing an biocompatible Co-Cr-Mo alloy, wherein the Co-Cr-Mo alloy powder according to claim 4 is layered-manufactured.

6. The process for producing an biocompatible Co-Cr-Mo alloy according to claim 5, wherein the layered manufacturing is carried out by irradiating laser of an output power of not less than 50 W and adjusting the scan pitch in the plane direction to be not less than 0.1 mm.

[Fig.1]

[Fig.2]

[Fig.3]

(a)

50 μm

(b)

50 μm

**INTERNATIONAL SEARCH REPORT**

| International application No. |
| --- |
| PCT/JP2012/076999 |

**A. CLASSIFICATION OF SUBJECT MATTER**
*C22C19/07*(2006.01)i, *B22F3/105*(2006.01)i, *B22F3/16*(2006.01)i, *C22C1/04*(2006.01)i

According to International Patent Classification (IPC) or to both national classification and IPC

**B. FIELDS SEARCHED**

Minimum documentation searched (classification system followed by classification symbols)
C22C19/07, B22F3/105, B22F3/16, C22C1/04

Documentation searched other than minimum documentation to the extent that such documents are included in the fields searched
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | Atsushi TAKAICHI, Keita YODA, Naoyuki NOMURA et al., "Application to dentistry of selective laser melting : Microstructures and mechanical properties of Co-Cr-Mo alloys fabricated by selective laser meltin", The Journal of the Japanese Society for Dental Materials and Devices, vol.29, no.5, 20 September 2010 (20.09.2010), page 445 | 1-6 |
| Y | Suyalatu, Naoyuki NOMURA, Atsushi TAKAICHI et al., "Microstructures and Mechanical Properties of Co-Cr-Mo Alloys Containing High Chromium and Nitrogen Fabricated by Selective Laser Melting Process", The Journal of the Japanese Society for Dental Materials and Devices, vol.30, no.5, 25 September 2011 (25.09.2011), page 292 | 1-6 |

| [X] Further documents are listed in the continuation of Box C. | [ ] See patent family annex. |
| --- | --- |

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 09 November, 2012 (09.11.12) | 20 November, 2012 (20.11.12) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

International application No.

PCT/JP2012/076999

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2010/026996 A1 (Tohoku University), 11 March 2010 (11.03.2010), claims; paragraphs [0006] to [0020]; example 2 & EP 2330227 A1 & CN 102131948 A | 1-6 |
| Y | JP 2009-114477 A (Iwate University), 28 May 2009 (28.05.2009), claims; paragraphs [0012] to [0026] (Family: none) | 1-6 |
| A | Yoshimi SATO, Naoyuki NOMURA, Shigeo FUJINUMA et al., "Microstructure and Tensile Properties of Hot-Pressed Co-Cr-Mo Alloy Compacts for Biomedical Applications", Journal of the Japan Institute of Metals, vol.72, no.7, 2008.07, pages 532 to 537 | 1-6 |
| A | Yoshimi SATO, Naoyuki NOMURA, Akihiko CHIBA, "Effect of Nitrogen Content on Microstructure of Hot-Pressed Co-Cr-Mo Alloy Compacts for Biomedical Applications", Journal of the Japan Institute of Metals, vol.72, no.11, 2008.11, pages 875 to 880 | 1-6 |
| A | Atsushi TAKAICHI, Suyalatu, Naoyuki NOMURA et al., "Anisotropic Mechanical Properties of Co-29Cr-6Mo Alloy Fabricated by Selective Laser Melting Process", The Journal of the Japanese Society for Dental Materials and Devices, vol. 30, no.5, 25 September 2011 (25.09.2011), page 293 | 1-6 |
| A | Naoyuki NOMURA, Keita YODA et al., "Effects of Cr and N of dental-cast Co-Cr-Mo alloys on their plastic deformation", The Journal of the Japanese Society for Dental Materials and Devices, vol.30, no.5, 25 September 2011 (25.09.2011), page 294 | 1-6 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009114477 A
- JP 4054075 B
- JP 2011231703 A